# FASCICULE DE BREVET EUROPEEN

(11) **EP 2 397 136 B1**
(45) Date de publication et mention de la délivrance du brevet: **28.03.2018**
(21) Numéro de dépôt: 11170436.7
(22) Date de dépôt: 17.06.2011
(51) Int. Cl.: A61K 31/35, A61K 31/593, A61K 45/06, A61K 36/185, A61P 19/02, A61P 29/00, A61K 31/352, A61K 33/06, A23L 33/155, A23L 33/105

(54) **Composition anti-inflammatoire**
Entzündungshemmende Zusammensetzung
Anti-inflammatory composition

(30) Priorité: 17.06.2010 FR 1054807
(43) Date de publication de la demande: 21.12.2011
(73) Titulaire: SP2L, 84000 Avignon (FR)
(72) Inventeur: Mathez, Laetitia, 13210 Saint-Rémy-de-Provence (FR)
(74) Mandataire: Icosa

(56) Documents cités:
- EP-A1- 1 371 372
- WO-A1-2010/037213
- CA-A1- 2 550 753
- DE-A1-102005 005 086
- FR-A1- 2 614 791
- GB-A- 2 335 919
- US-A- 5 888 514
- US-A1- 2002 183 264
- US-A1- 2005 080 059
- INABA K ET AL: "Inhibitory effects of devil's claw (secondary root of Harpagophytum procumbens) extract and harpagoside on cytokine production in mouse macrophages", JOURNAL OF NATURAL MEDICINES 2010 SPRINGER JAPAN JPN LNKD- DOI:10.1007/S11418-010-0395-8, vol. 64, no. 2, avril 2010 (2010-04), pages 219-222, XP002661056, ISSN: 1340-3443
- ALAPPAT L ET AL: "Effect of vitamin D and beta-sitosterol on immune function of macrophages", INTERNATIONAL IMMUNOPHARMACOLOGY, ELSEVIER, AMSTERDAM, NL, vol. 10, no. 11, 1 novembre 2010 (2010-11-01), pages 1390-1396, XP027430062, ISSN: 1567-5769, DOI: 10.1016/J.INTIMP.2010.08.003 [extrait le 2010-08-20]

## Description

La présente invention concerne une nouvelle composition nutritionnelle ayant des propriétés anti-inflammatoires. Plus particulièrement, cette composition est utile pour la prévention ou le traitement de l'inflammation et notamment de la douleur induite par l'inflammation. Enfin, la composition selon l'invention est en particulier utile pour la prévention et le traitement de l'inflammation des articulations, l'ostéoarthrite, et d'autres maladies dégénératives des articulations.

L'inflammation est caractérisée comme un type de réponse immune non spécifique mise en oeuvre par l'organisme en réaction à une infection, une irritation ou une blessure. Les symptômes typiques de l'inflammation sont la rougeur, la sensation de chaleur, le gonflement et la douleur. De manière générale, l'inflammation est fortement régulée par l'organisme. Cependant, lorsque les moyens physiologiques de contrôle de l'inflammation sont inopérants ou dépassés, l'inflammation se développe et peut devenir la cause de certaines maladies telles que l'ostéoarthrite, l'athérosclérose, les maladies inflammatoires de l'intestin ou l'arthrite rhumatoïde.
Des millions de personnes souffrent de la douleur induite par l'inflammation de tissus conjonctifs, notamment de l'inflammation des articulations. Le terme arthrite se réfère aux conditions dans lesquelles une inflammation des articulations peut être observée. Les symptômes caractéristiques d'une inflammation des articulations sont : une douleur au niveau des tendons enflammés, à l'effort (seulement en début d'effort, puis disparaissant avec l'échauffement en cas d'inflammation légère), des élancements même au repos ou une douleur chronique dans les cas plus graves, avec éventuellement : une inflammation (rougeur, sensation de chaleur) et un gonflement de l'articulation (bursite) et enfin une mobilité réduite.
L'inflammation des articulations est également présente dans l'ostéoarthrite, commune chez les personnes âgées.
A l'heure actuelle, les maladies inflammatoires sont généralement traitées par des médicaments anti-inflammatoires stéroïdiens ou non stéroïdiens. Les composés stéroïdiens sont basés sur l'utilisation de substances hormonales telles que la cortisone. Les composés non stéroïdiens sont les plus couramment utilisés, comme par exemple l'acétaminophène, l'aspirine, l'ibuprofène, ou le naproxène. Ces médicaments provoquent la plupart du temps des effets secondaires non désirables, tels que des brulures d'estomac, des hémorragies digestives, des ulcères, des insuffisances rénales, une hypertension artérielle ou encore des problèmes d'audition. De plus, les médicaments stéroïdiens ont montré d'autres effets secondaires non désirables tels que des troubles métaboliques (ostéoporose, hypokaliémie, alcalose métabolique, retard de croissance chez l'enfant, retard de cicatrisation) ou encore des troubles psychiques (excitation, confusion, dépression).
En marge de ces traitements palliatifs ou d'opérations chirurgicales lourdes (pose de prothèses...), sont récemment apparues des compositions de type médicaments, compléments alimentaires ou alicaments, élaborées pour prévenir et/ou traiter l'inflammation. En effet, les compositions à base de plantes sont en général bien tolérées par l'organisme et n'induisent pas d'effets secondaires sévères. Les compositions à base de plantes présentent également l'avantage d'exercer leur effet bénéfique via une action additive ou synergique de différents composés chimiques agissant sur de multiples sites cibles associés à une réponse physiologique.

La Demanderesse s'est ainsi penchée sur le développement d'une composition à base de plantes, qui procure moins d'effets secondaires lorsqu'elle est administrée quotidiennement par rapport aux traitements existants, pour la prévention et/ou le traitement de l'inflammation. Lors de ses recherches, la Demanderesse a identifié que la combinaison spécifique d'Harpagophytum et de vitamine D présentait un avantage pour le traitement de l'inflammation car ces deux composés agissent en synergie. Le terme « synergie » tel qu'utilisé dans la présente invention se réfère à un phénomène dans lequel la combinaison de deux ou plus agents agissant ensemble induit une réponse plus importante que la somme des réponses de chaque agent pris individuellement
Des compositions comprenant de l'Harpagophytum ont déjà été décrites et l'Harpagophytum est connu pour son utilisation dans le traitement de la douleur. Par exemple, la demande de brevet FR2829692 décrit la Formulation N°3 comprenant entre autres de l'Harpagophytum avec un mélange de vitamines (vitamines B1, B3, B6, B9 et C) pour la régulation des états douloureux chroniques.
La présente invention a donc pour objet une composition comprenant de l'Harpagophytum ou de l'harpagoside et de la vitamine D.

L'Harpagophytum est une plante de la famille des Pedaliaceae, dont la principale espèce est Harpagophytum procumbens communément appelée « griffe du diable » ou « racine de Windhoek ». Une autre espèce connue d'Harpagophytum est l'Harpagophytum zeyheri. L'Harpagophytum est une herbacée vivace originaire des régions semi-désertiques sud africaines.
L'Harpagophytum est une plante médicinale dont la racine est inscrite dans la pharmacopée européenne pour le traitement de douleurs articulaires mineures. Les deux principes actifs de l'Harpagophytum sont les harpagosides, notamment l'harpagoside, et le beta-sitostérol. Ces molécules possèdent des propriétés anti-inflammatoires. Les racines d'Harpagophytum contiennent également des flavonoïdes, des acides phénoliques, des quinones, des phytostérols, des sucres, des triterpènes et des acétosides.

Dans un mode de réalisation de l'invention, la composition selon l'invention comprend l'Harpagophytum sous forme de poudre ou sous forme d'extrait.
Les racines secondaires d'Harpagophytum peuvent être utilisées pharmaceutiquement et peuvent être préparées sous forme de thé, sous forme de poudre ou subir une extraction pour l'obtention d'un extrait sec ou liquide.
Des extraits d'Harpagophytum peuvent être préparés par toutes techniques bien connues de l'homme du métier, comme par exemple l'extraction par entrainement à la vapeur (hydrodistillation). De préférence, l'extrait d'Harpagophytum est obtenu par extraction à partir de la plante Harpagophytum, en particulier par hydrodistillation ou par extraction hydro-alcoolique, de préférence éthanol ou méthanol/eau. L'avantage d'utiliser de l'eau comme milieu d'extraction est que le produit peut directement être incorporé dans le produit final sans avoir à éliminer le milieu d'extraction préalablement.

Un autre procédé de production d'extraits d'Harpagophytum est décrit dans la demande de brevet WO2008/145931 dont le contenu est incorporé à la présente demande. Ce procédé de préparation comprend une étape de purification d'un extrait brut d'Harpagophytum sous forme liquide en phase gazeuse, par une technique d'extraction liquide-liquide avec un solvant organique choisi parmi les esters, de préférence l'acétate d'alkyle, de façon à obtenir une phase aqueuse et une phase organique concentrée en harpagoside. Le solvant est ensuite éliminé pour obtenir l'extrait concentré sous forme liquide et ledit extrait est optionnellement transformé pour obtenir un extrait sous forme sèche. Les méthodes bien connues de l'homme de l'art pour transformer un extrait concentré sous forme liquide en extrait sous forme sèche sont l'élimination de l'eau par flux d'air chaud, par séchage, par nébulisation, par évaporation, par sublimation, par déshydratation ou par adsorption sur un support.
De manière générale, les extraits d'Harpagophytum comprennent 0,5 à 20 % d'harpagosides, mesuré par HPLC. Ces extraits offrent une alternative satisfaisante à l'usage de poudre de plante.
Le procédé décrit dans WO2008/145931 permet d'obtenir des extraits d'Harpagophytum sous forme liquide ou sèche, comprenant une concentration en harpagosides supérieure ou égale à 5%, voire supérieure ou égale à 35%.
Des extraits susceptibles d'être utilisés dans la présente invention sont par exemple les extraits commercialisés par Naturex sous le nom de Devil's Claw, ou les extraits commercialisés par Burgundy sous le nom Botany Harpagophytum (5% harpagosides) ou BotanySelect Harpagophytum (20% harpagosides).

Dans un mode de réalisation de l'invention, la composition selon l'invention comprend 100 à 3000 mg de poudre d'Harpagophytum, de préférence 200 à 2000 mg, plus préférentiellement de 400 à 1500 mg, encore plus préférentiellement de 600 à 900 mg et enfin encore préférentiellement 800 mg de poudre d'Harpagophytum.

Dans un autre mode de réalisation de l'invention, la composition selon l'invention comprend 50 à 3000 mg d'extrait d'Harpagophytum contenant 0,5 à 5% d'harpagosides, de préférence 100 à 1000 mg, plus préférentiellement de 150 à 700 mg, encore plus préférentiellement de 200 à 500 mg. Dans un mode de réalisation, la composition selon l'invention comprend 400 mg d'extrait d'Harpagophytum contenant 0,5 à 5% d'harpagosides. Dans un autre mode de réalisation, la composition selon l'invention comprend 250 mg d'extrait d'Harpagophytum contenant 0,5 à 5% d'harpagosides.

Dans un autre mode de réalisation de l'invention, la composition selon l'invention comprend 50 à 1500 mg d'extrait d'Harpagophytum contenant 10% d'harpagosides, de préférence 100 à 1000 mg, plus préférentiellement de 125 à 400 mg, encore plus préférentiellement 150 à 300 mg. Dans un mode de réalisation, la composition selon l'invention comprend 200 mg d'extrait d'Harpagophytum contenant 10% d'harpagosides. Dans un autre mode de réalisation, la composition selon l'invention comprend 400 mg d'extrait d'Harpagophytum contenant 10% d'harpagosides.

Dans un autre mode de réalisation de l'invention, la composition selon l'invention comprend 25 à 750 mg d'extrait d'Harpagophytum contenant 20% d'harpagosides, de préférence 50 à 500 mg, plus préférentiellement de 60 à 200 mg. encore plus préférentiellement 75 à 150 mg. Dans un mode de réalisation, la composition selon l'invention comprend 100 mg d'extrait d'Harpagophytum contenant 20% d'harpagosides. Dans un autre mode de réalisation, la composition selon l'invention comprend 200 mg d'extrait d'Harpagophytum contenant 20% d'harpagosides.

Dans un autre mode de réalisation de l'invention, la composition selon l'invention comprend 15 à 500 mg d'extrait d'Harpagophytum contenant 30% d'harpagosides, de préférence 30 à 300 mg, plus préférentiellement de 40 à 130 mg, encore plus préférentiellement 50 à 100 mg. Dans un mode de réalisation, la composition selon l'invention comprend 60 mg d'extrait d'Harpagophytum contenant 30% d'harpagosides. Dans un autre mode de réalisation, la composition selon l'invention comprend 120 mg d'extrait d'Harpagophytum contenant 30% d'harpagosides.

Dans un autre mode de réalisation de l'invention, la composition selon l'invention comprend 12,5 à 375 mg d'extrait d'Harpagophytum contenant 40% d'harpagosides, de préférence 25 à 250 mg, plus préférentiellement de 30 à 100 mg, encore plus préférentiellement 35 à 75 mg. Dans un mode de réalisation, la composition selon l'invention comprend 50 mg d'extrait d'Harpagophytum contenant 40% d'harpagosides. Dans un autre mode de réalisation, la composition selon l'invention comprend 100 mg d'extrait d'Harpagophytum contenant 40% d'harpagosides.

Dans un mode de réalisation de l'invention, la composition selon l'invention comprend de 1 à 200 mg d'harpagosides, de 1 à 100 mg, de préférence 10 à 50 mg, plus préférentiellement de 15 à 30 mg d'harpagosides. Dans un mode de réalisation, la composition selon l'invention comprend 40 mg d'harpagosides. Dans un mode de réalisation, la composition selon l'invention comprend 1.25 mg d'harpagosides.
Dans un mode de réalisation de l'invention, la composition selon l'invention comprend de 0.1 à 100 mg d'harpagoside, de 0.25 à 50 mg, de préférence 0.5 à 25 mg, plus préférentiellement de 0.6 à 12.5 mg d'harpagoside. Dans un mode de réalisation, la composition selon l'invention comprend 20 mg d'harpagoside. Dans un mode de réalisation, la composition selon l'invention comprend 0.625 mg d'harpagoside.

La Vitamine D est une vitamine liposoluble synthétisée par l'organisme humain à partir d'un dérivé du cholestérol sous l'action des rayonnements UVB de la lumière. Elle existe sous deux formes : la vitamine D2 (ergocalciférol) et la vitamine D3 (cholécalciférol).

Dans un mode de réalisation de l'invention, la composition selon l'invention comprend de la vitamine D2 ou de la vitamine D3 ou un mélange des deux.
De préférence, la composition selon l'invention comprend de la vitamine D3.
La vitamine D est disponible commercialement.

Dans un mode de réalisation de l'invention, la composition selon l'invention comprend 0,5 µg à 50 µg de vitamine D, de préférence 1 µg à 25 µg, plus préférentiellement de 5 µg à 15 µg et encore plus préférentiellement 5 µg à 10 µg de vitamine D.

Dans un mode de réalisation de l'invention, la composition comprend de l'Harpagophytum ou des harpagosides et de la vitamine D, et la quantité d'Harpagophytum est de 50 à 3000 mg, la quantité d'harpagosides est de 1 à 100 mg et la quantité de vitamine D est de 0.5 à 50 µg.

Dans un mode de réalisation de l'invention, la composition comprend de la poudre d'Harpagophytum, ou un extrait d'Harpagophytum à 20% d'harpagosides ou des harpagosides et de la vitamine D, et la quantité de poudre d'Harpagophytum est de 2400 mg, la quantité d'extrait d'Harpagophytum est de 200 mg, la quantité d'harpagosides est de 40 mg et la quantité de vitamine D est de 5 µg.

Dans un mode de réalisation de l'invention, la composition comprend de la poudre d'Harpagophytum, ou un extrait d'Harpagophytum à 0.5% d'harpagosides ou des harpagosides et de la vitamine D, et la quantité de poudre d'Harpagophytum est de 1000 mg, la quantité d'extrait d'Harpagophytum est de 250 mg, la quantité d'harpagosides est de 1.25 mg et la quantité de vitamine D est de 5 µg.

Dans un mode de réalisation de l'invention, la composition selon l'invention comprend un ratio d'Harpagophytum (extrait ou poudre) : vitamine D de 6 000 000 :1 à 1000 :1, de préférence de 1 000 000:1 à 10 000 :1, plus préférentiellement de 500 000 :1 à 25 000 :1, et encore plus préférentiellement de 100 000 :1 à 40 000 :1.
Dans un mode de réalisation de l'invention, la composition selon l'invention comprend un ratio d'harpagosides: vitamine D de 200 000 :1 à 20 :1, de préférence de 50000 :1 à 200 :1, de préférence de 50000 :1 à 400 :1, de préférence de 10 000 :1 à 500 :1, plus préférentiellement de 6000 :1 à 1000 :1, et encore plus préférentiellement de 4000 :1 à 2000 :1.
Dans un mode de réalisation de l'invention, ces ratios sont en moles de produit. Dans un autre mode de réalisation, ces ratios sont en poids de produit.

Dans un mode de réalisation de l'invention, la composition selon l'invention comprend également du calcium.
Selon l'invention, le calcium peut être choisi parmi les composés suivants :
carbonate de calcium, chlorure de calcium, malate de citrate de calcium, sels calciques de l'acide citrique, gluconate de calcium, glycérophosphate de calcium, lactate de calcium, sels calciques de l'acide orthophosphorique, hydroxyde de calcium, malate de calcium, oxyde de calcium, sulfate de calcium, acétate de calcium, L-ascorbate de calcium, bisglycinate de calcium, pyruvate de calcium, succinate de calcium, L-lysinate de calcium, L-pidolate de calcium, et L-thréonate de calcium.
De préférence, la composition selon l'invention comprend du carbonate de calcium.
Le calcium est disponible commercialement auprès de très nombreux fournisseurs.

Dans un mode de réalisation de l'invention, la composition selon l'invention comprend 100 mg à 2000 mg de calcium, de préférence 200 mg à 1000 mg, plus préférentiellement de 300 mg à 500 mg et encore plus préférentiellement 400 mg de calcium.

De manière préférée, la composition selon l'invention comprend 800 mg de poudre d'Harpagophytum et 5 µg de vitamine D, et optionnellement 400 mg de calcium.
De manière préférée, la composition selon l'invention comprend 400 mg d'extrait d'Harpagophytum contenant 0,5 à 5% d'harpagosides et 5 µg de vitamine D, et optionnellement 400 mg de calcium.
De manière préférée, la composition selon l'invention comprend 200 mg d'extrait d'Harpagophytum contenant 10% d'harpagosides et 5 µg de vitamine D, et optionnellement 400 mg de calcium.
De manière préférée, la composition selon l'invention comprend 100 mg d'extrait d'Harpagophytum contenant 20% d'harpagosides et 5 µg de vitamine D, et optionnellement 400 mg de calcium.
De manière préférée, la composition selon l'invention comprend 60 mg d'extrait d'Harpagophytum contenant 30% d'harpagosides et 5 µg de vitamine D, et optionnellement 400 mg de calcium.
De manière préférée, la composition selon l'invention comprend 50 mg d'extrait d'Harpagophytum contenant 40% d'harpagosides et 5 µg de vitamine D, et optionnellement 400 mg de calcium.
De manière préférée, la composition selon l'invention comprend 20 mg d'harpagosides et 5 µg de vitamine D, et optionnellement 400 mg de calcium.

Dans un mode de réalisation de l'invention, la composition selon l'invention peut être un produit alimentaire, une boisson, un complément alimentaire, un produit nutraceutique ou alicament, un produit cosmétique, un produit d'alimentation vétérinaire ou un médicament.

Dans un mode de réalisation de l'invention, la composition selon l'invention comprend optionnellement un ou plusieurs excipients pharmaceutiquement ou nutraceutiquement acceptables ou des sels ou des additifs.

Dans un mode de réalisation de l'invention, la composition selon l'invention est sous la forme de granules, comprimés, capsules, gélules, solution, suspension, sirop, poudre, pâte, gel, ou crème.

Dans un mode de réalisation de l'invention, la composition selon l'invention est formulée sous une forme de dosage solide, semi-solide ou liquide par addition de véhicules biologiquement ou pharmaceutiquement ou nutraceutiquement acceptables.

Les produits selon l'invention sont administrés de préférence par voie entérale ou par voie topique. Une administration parentérale peut également être envisagée. On entend par voie entérale la voie orale, la voie sublinguale, la voie rectale, la voie pulmonaire, la voie percutanée et les voies locales.

Dans un mode de réalisation de l'invention, des formes de dosage spécifiques pour la formulation de la composition selon l'invention incluent, mais ne sont pas limitées à, des formulations orales telles que des granules, des comprimés, des capsules souples ou dures, des poudres, des solutions pour infusion, des crèmes, des gels, des émulsions (émulsion huile dans eau, eau dans huile, anhydre, solide ou microémulsions), des onguents, des lavements, des suspensions, des sirops, des ampoules, des inhalateurs, des sprays pour la bouche, des injections, des gouttes, des suppositoires, des patches...

Dans un mode de réalisation de l'invention, la forme galénique comprenant la composition de l'invention est unique, i.e. une seule gélule ou une seule formulation liquide.

Des exemples de véhicules biologiquement ou pharmaceutiquement ou nutraceutiquement acceptables incluent, mais ne sont pas limités à, des surfactants, des excipients, des liants, des diluants, des lubrifiants, des conservateurs, des stabilisateurs, des antioxydants, des tampons, des suspensions et des systèmes d'administration.
Des exemples de véhicules, diluants ou excipients solides incluent, mais ne sont pas limités au glucose, fructose, sucrose, maltose, dextrine jaune, dextrine blanche, cellulose microcristalline, stéarate de calcium, stéarate de magnésium, sorbitol, sirop de glucose, lactose, acide citrique, acide tartrique, acide malique, acide succinique, acide lactique, acide L-ascorbique, alpha-tocophérol, glycérine, propylène glycol, le sucroester, les esters polyglycériques d'acides gras, les sucroglycérides, les carraghénanes, la gomme arabique, la caséine, la gélatine, la pectine, l'agar, la nicotinamide, les acides aminés, les sels de calciums, les pigments...
Des exemples de véhicules liquides incluent de l'eau distillée, une solution saline, une solution aqueuse de glucose, de l'alcool par exemple de l'éthanol, du propylène glycol, et de polyéthylène glycol ; et des véhicules huileux tels que des huiles végétales et animales, de la paraffine, ou de la cire.
Des exemples d'antioxydants incluent mais ne sont pas limités à du tocophérol, du butylhydroxytoluène (BHT), du butylhydroxyanisol (BHA), des antioxydants naturels comme l'extrait de romarin, du gallate de propyle...
Des exemples de conservateurs incluent mais ne sont pas limités à du méthylparabène, du propylparabène, du sorbate de potassium, du benzoate de sodium, de l'acide benzoïque... Des exemples de surfactants incluent mais ne sont pas limités à des surfactants anioniques, cationiques, ou non-ioniques tels que le palmitate d'ascorbyle, les polysorbates, les polyéthylènes glycols...
Des exemples d'agents stabilisateurs du pH ou des tampons incluent mais ne sont pas limités à acide citrique-citrate de sodium, acide phosphorique-phosphate de sodium, acide acétique-acétate de sodium...

Dans un autre mode de réalisation de l'invention, les compositions selon l'invention sont administrées sous forme de comprimés à libération contrôlée, utilisant des revêtements à base de polymères permettant la libération contrôlée grâce à des techniques bien connues de l'homme du métier telles que la micro-encaspulation ou des systèmes de véhicule colloïdal.
Des exemples d'agents d'encapsulation incluent, mais ne sont pas limités à, de l'amidon, des protéines de source animale comme la gélatine, des protéines de source végétale, de la caséine, de la pectine, d'alginate, de l'agar, des maltodextrines, des sulfonates de lignine, des dérivés cellulosiques (éthylcellulose, méthylcellulose, hydroxypropylcellulose, hydroxypropylméthylcellulose, carboxyméthylcellulose), des sucres, sorbitols, gommes....

Les compositions selon l'invention sont de préférence à destination des humains mais peuvent également être administrées à des animaux, notamment aux animaux domestiques.

Dans un mode de réalisation de l'invention, la composition selon l'invention peut être comprise dans des produits alimentaires ou des boissons tels que du thé, des sucreries, des produits à usage culinaire, des compléments nutritionnels, des produits laitiers, des boissons, des boissons à base de lait, des soupes, des substituts de repas, des barres nutritionnelles, des poudres à base de lait, des produits céréaliers, des biscuits, des gommes à mâcher, du chocolat...

Dans un autre mode de réalisation de l'invention, la composition selon l'invention peut être comprise dans des produits cosmétiques tels que du savon, du shampoing, du dentifrice, une solution rince-bouche, du rouge à lèvre, des crèmes...

Dans un mode de réalisation de l'invention, la composition selon l'invention permet la prévention et/ou le traitement de l'inflammation.

Selon l'invention, le terme «prévention» se réfère à l'action de la composition selon l'invention d'empêcher ou de retarder ou de limiter le développement de l'inflammation. Le terme «traitement» selon l'invention se réfère à l'action de la composition selon l'invention de diminuer, de supprimer ou d'inhiber les symptômes liés à l'inflammation, de ralentir la progression de l'inflammation, d'améliorer la condition des sujets auxquels est administré la composition.

Dans un mode de réalisation de l'invention, la composition selon l'invention permet la prévention et/ou le traitement de maladies inflammatoires.

L'invention a également pour objet une méthode de traitement de l'inflammation ou des maladies inflammatoires, comprenant l'administration à un sujet d'une quantité thérapeutique suffisante de la composition de l'invention.
Des exemples de ces maladies incluent mais ne sont pas limités à l'asthme, l'arthrite, athérosclérose, les dysfonctions endothéliales, l'ostéoarthrite, l'arthrite rhumatoïde, les rhinites allergiques, les dermatites, le psoriasis, les fibroses cystiques, les maladies inflammatoires de l'intestin, la sclérose en plaque, le diabète, les désordres neurologiques, les lupus, les resténoses, les glomérulonéphrites, les allergies gastrointestinales, les néphrites, les conjonctivites, l'eczéma, les bronchites, le rhume des foins, les douleurs articulaires, le syndrome de Sjögren, la sclérodermie, la dermatomyosite, la polymyosite, la polypyalgia rheumatica, la goutte, les spondylarthropathies, les vascularites...

Dans un mode de réalisation préféré de l'invention, la composition selon l'invention permet la prévention et/ou le traitement de la douleur induite par l'inflammation.

Dans un autre mode de réalisation préféré de l'invention, la composition selon l'invention permet la prévention et/ou le traitement des maladies dégénératives des articulations, notamment de l'arthrose.

Dans un mode de réalisation de l'invention, la composition selon l'invention est administrée quotidiennement au sujet.

Dans un mode de réalisation de l'invention, la composition selon l'invention est administrée une fois par jour au sujet.

Dans un mode de réalisation de l'invention, la composition selon l'invention est administrée deux fois par jour au sujet.

Dans un mode de réalisation de l'invention, la composition selon l'invention est administrée trois fois par jour au sujet.

### Exemples

### Exemple 1

Une personne souffrant de rhumatismes des genoux a pris quotidiennement pendant plusieurs mois une gélule contenant de la poudre d'Harpagophytum (poudre de racine contenant 3% d'harpagosides).

Elle a constaté que la sensation de raideur articulaire au niveau des genoux est légèrement atténuée.

Cette personne a ensuite pris simultanément des gélules contenant de la poudre d'Harpagophytum et des comprimés de vitamine D sous forme de Cholécalciférol (25mcg) pendant plusieurs semaines.

Elle a constaté une amélioration importante de la sensation de raideur et de douleur au niveau des genoux. Cette nette amélioration s'est poursuivie au cours des semaines de traitement.

### Exemple 2

Trois personnes souffrant de rhumatismes ont pris simultanément des gélules contenant de la poudre d'Harpagophytum et des comprimés de vitamine D sous forme de Cholécalciférol pendant plusieurs semaines : ces trois personnes ont également observé une nette amélioration de la douleur et de la sensation de raideur au niveau de leurs articulations.

### Exemple 3

Une personne souffrant de rhumatismes des hanches a pris simultanément des gélules contenant de la poudre d'Harpagophytum et des comprimés de vitamine D sous forme de Cholécalciférol (800 UI soit 20 mcg) pendant plusieurs semaines : elle a remarqué une nette amélioration de sa douleur au niveau des hanches et une diminution dans la raideur de ses articulations.

Elle a ensuite ajouté à ce traitement du calcium (1000 mg par jour sous forme de carbonate de calcium) pendant plusieurs semaines et a observé une encore meilleure amélioration dans sa douleur.

### Exemple 4

### Matériels et Méthodes

### Culture cellulaire

Les cellules RAW 264.7 sont ensemencées en plaque 48 puits et incubées une nuit à 37°C, 5% CO2. Le lendemain, les cellules sont prétraitées 1 heure par les produits à tester et leurs différentes combinaisons, puis l'inflammation est induite par le LPS à 1Fg/ml. Après 24 heures d'incubation, les surnageants cellulaires sont récupérés puis congelés à -20°C.

### Dosage de l'IL1-β par ELISA

Le dosage de la cytokine IL1-β est réalisé à partir de surnageants de culture par la technique d'ELISA et à l'aide d'un kit commercial (*Mouse IL1β ELISA Set, 559603, BD Biosciences)* selon les instructions du fournisseur.

### Analyse des données

Analyse des dosages de cytokines : Le contrôle positif d'inflammation est représenté par les cellules incubées uniquement avec le LPS. Le contrôle négatif d'inflammation de l'expérience n'est pas stimulé par le LPS. Enfin, la Dexamethasone est utilisée comme témoin positif d'inhibition de l'inflammation.

La sécrétion des cytokines est présentée en pourcentage d'inhibition de l'inflammation par rapport aux cellules stimulées par le LPS pendant 24 heures.

### Produits testés

Un extrait d'Harpagophytum dosé à 10% d'harpagosides est utilisé à une concentration en harpagosides de 0.2 µM, 2 µM et 8 µM.

Du calcitriol (1α,25 (OH)2 D3) est utilisé à une concentration de 1 nM ou de 10 nM.

### Résultats

Les pourcentages d'inhibition de la sécrétion d'IL-1β par les harpagosides, le calcitriol ou leur combinaison sont représentés dans le tableau ci-dessous.

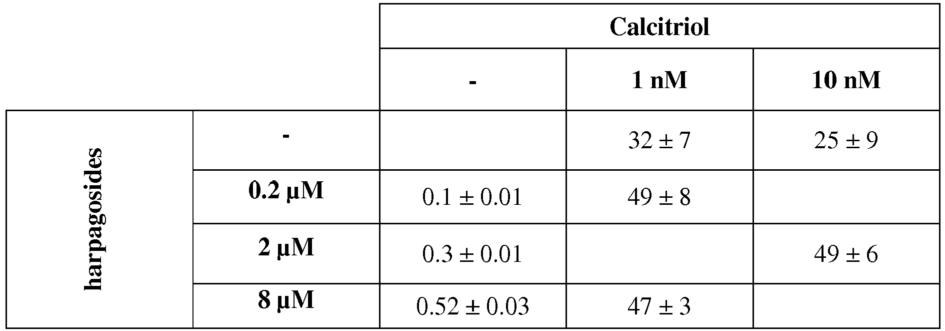

Il existe donc un effet synergique des harpagosides et de la vitamine D sur l'inhibition de la sécrétion d'IL-1β. La cytokine IL-1β est une cytokine inflammatoire : une inhibition de la sécrétion d'IL-1β indique donc un effet anti-inflammatoire.

L'Harpagophytum et la vitamine D ont des actions anti-inflammatoires synergiques.

## Revendications

1. Composition comprenant de l'Harpagophytum ou des harpagosides et de la vitamine D, dans laquelle la quantité d'Harpagophytum est de 50 à 3000 mg, la quantité d'harpagosides est de 1 à 100 mg et la quantité de vitamine D est de 0.5 à 50 µg.

2. Une composition selon la revendication **1**, dans laquelle l'Harpagophytum est sous forme de poudre ou sous forme d'extrait.

3. Une composition selon la revendication **1** ou **2**, comprenant de 100 à 3000 mg de poudre d'Harpagophytum.

4. Une composition selon la revendication **1** ou **2**, comprenant de 50 à 500 mg d'extrait d'Harpagophytum contenant 10 à 40% d'harpagosides.

5. Une composition selon la revendication **1** ou **2**, comprenant de 50 à 3000 mg d'extrait d'Harpagophytum contenant 0,5 à 5% d'harpagosides.

6. Une composition selon l'une quelconque des revendications **1** à **5**, comprenant de la poudre d'Harpagophytum dans une quantité de 2400 mg, ou un extrait d'Harpagophytum à 20% d'harpagosides dans une quantité de 200 mg, ou des harpagosides dans une quantité de 40 mg et de la vitamine D dans une quantité de 5 µg.

7. Une composition selon l'une quelconque des revendications **1** à **5**, comprenant de la poudre d'Harpagophytum dans une quantité de 1000 mg, ou un extrait d'Harpagophytum à 0.5% d'harpagosides dans une quantité de 250 mg, ou des harpagosides dans une quantité de 1.25 mg et de la vitamine D dans une quantité de 5 µg.

8. Une composition selon l'une quelconque des revendications **1** à **7**, dans laquelle la vitamine D est sous la forme d'ergocalciférol, de cholécalciférol ou d'un mélange des deux.

9. Une composition selon l'une quelconque des revendications **1** à **8**, comprenant en outre du calcium.

10. Une composition selon l'une quelconque des revendications **1** à **9**, sous la forme d'un produit alimentaire, d'une boisson, d'un complément alimentaire, d'un produit nutraceutique ou d'alicament, d'un produit cosmétique, d'un produit d'alimentation vétérinaire ou d'un médicament.

11. Une composition selon l'une quelconque des revendications **1** à **10**, sous la forme de granules, comprimés, capsules, gélules, solution, suspension, sirop, poudre, pâte, gel, ou crème.

12. Une composition selon l'une quelconque des revendications **1** à **11** pour son utilisation dans la prévention et/ou le traitement de l'inflammation ou de maladies inflammatoires.

13. Une composition pour son utilisation selon la revendication **12**, pour la prévention et/ou le traitement de la douleur induite par l'inflammation.

14. Une composition pour son utilisation selon la revendication **12**, pour la prévention et/ou le traitement des maladies dégénératives des articulations, notamment de l'arthrose.

## Patentansprüche

1. Zusammensetzung, die Harpagophytum oder Harpagoside und Vitamin-D umfasst, wobei die Menge an Harpagophytum reicht von 50 bis 3000 mg, die Menge an Harpagosiden reicht von 1 bis 100 mg und die Menge an Vitamin-D reicht von 0,5 bis 50 µg.

2. Zusammensetzung nach Anspruch **1**, wobei das Harpagophytum in Pulverform oder Extraktform ist.

3. Zusammensetzung nach Anspruch **1** oder **2**, die von 100 bis 3000 mg Harpagophytum-Pulver umfasst.

4. Zusammensetzung nach Anspruch **1** oder **2**, die von 50 bis 500 mg Harpagophytum-Extrakt umfasst, der 10 bis 40% Harpagoside enthält.

5. Zusammensetzung nach Anspruch **1** oder **2**, die von 50 bis 3000 mg Harpagophytum-Extrakt umfasst, der 0,5 bis 5% Harpagoside enthält.

6. Zusammensetzung nach einem der Ansprüche **1** bis **5**, die Harpagophytum-Pulver in einer Menge von 2400 mg, oder einen Harpagophytum-Extrakt mit 20% Harpagosiden in einer Menge von 200 mg, oder Harpagoside in einer Menge von 40 mg und Vitamin-D in einer Menge von 5 µg umfasst.

7. Zusammensetzung nach einem der Ansprüche **1** bis **5**, die Harpagophytum-Pulver in einer Menge von 1000 mg, oder einen Harpagophytum-Extrakt mit 0,5% Harpagosiden in einer Menge von 250 mg, oder Harpagoside in einer Menge von 1,25 mg und Vitamin-D in einer Menge von 5 µg umfasst.

8. Zusammensetzung nach einem der Ansprüche **1** bis **7**, wobei das Vitamin-D in der Form von Ergocalciferol, Cholecalciferol oder einem Gemisch der beiden ist.

9. Zusammensetzung nach einem der Ansprüche **1** bis **8**, die außerdem Calcium umfasst.

10. Zusammensetzung nach einem der Ansprüche **1** bis **9**, in der Form eines Nahrungsmittelprodukts, eines Getränks, eines Nahrungsergänzungsmittels, eines Neutraceuticals oder Functional Foods, eines kosmetischen Produkts, eines Tierfutterprodukts oder eines Arzneimittels.

11. Zusammensetzung nach einem der Ansprüche **1** bis **10**, in der Form von Granulat, Tabletten, Kapseln, Gelkapseln, einer Lösung, einer Suspension, eines Sirups, eines Pulvers, einer Paste, eines Gels oder einer Creme.

12. Zusammensetzung nach einem der Ansprüche **1** bis **11** zu deren Verwendung bei der Verhinderung und/oder Behandlung einer Entzündung oder von Entzündungserkrankungen.

13. Zusammensetzung zu deren Verwendung nach Anspruch **12,** bei der Verhinderung und/oder Behandlung von durch eine Entzündung hervorgerufene Schmerzen.

14. Zusammensetzung zu deren Verwendung nach Anspruch **12**, bei der Verhinderung und/oder Behandlung von degenerativen Gelenkerkrankungen, insbesondere Arthrose.

## Claims

1. A composition comprising Harpagophytum or harpagosides and vitamin D, wherein the amount of Harpagophytum ranges from 50 to 3000 mg, the amount of harpagosides ranges from 1 to 100 mg and amount of vitamin D ranges from 0.5 to 50 µg.

2. The composition according to claim **1**, wherein Harpagophytum is in the form of powder or in the form of extract.

3. The composition according to claim **1** or **2**, comprising from 100 to 3000 mg of Harpagophytum powder.

4. The composition according to claim **1** or **2**, comprising from 50 to 500 mg of Harpagophytum extract containing 10 to 40% of harpagosides.

5. The composition according to claim **1** or **2**, comprising from 50 to 3000 mg of Harpagophytum extract containing 0.5 to 5% of harpagosides.

6. The composition according to any one of claims **1** to **5**, comprising Harpagophytum powder in an amount of 2400 mg, or a 20% harpagosides Harpagophytum extract in an amount of 200 mg, or harpagosides in an amount of 40 mg and vitamin D in an amount of 5 µg.

7. The composition according to any one of claims **1** to **5**, comprising Harpagophytum powder in an amount of 1000 mg, or a 0.5% harpagosides Harpagophytum extract in an amount of 250 mg, or harpagosides in an amount of 1.25 mg and vitamin D in an amount of 5 µg.

8. The composition according any one of claims **1** to **7**, wherein vitamin D is in the form of ergocalciferol, cholecalciferol or a mix of both.

9. The composition according to any one of claims **1** to **8**, further comprising calcium.

10. The composition according any one of claims **1** to **9**, in the form of a food product, a beverage, a dietary supplement, a nutraceutical product or a functional food, a cosmetic product, a veterinary food product or a drug.

11. The composition according to any one of claims **1** to **10**, in the form of granules, tablets, capsules, caplets, solution, suspension, syrup, powder, paste, gel, or cream.

12. The composition according to any one of claims **1** to **11** for use in the prevention and/or treatment of inflammation or inflammatory diseases.

13. The composition for use according to claim **12**, for preventing and/or treating inflammation-induced pain.

14. The composition for use according to claim **12**, for preventing and/or treating degenerative joint diseases, including osteoarthritis.
